Europäisches Patentamt

European Patent Office

Office européen des brevets

(19)

(11) Publication number: **0 393 228**
**A1**

## (12) EUROPEAN PATENT APPLICATION

(21) Application number: **89107263.9**

(22) Date of filing: **21.04.89**

(51) Int. Cl.5: **A61B 5/02, G06F 15/20**

(43) Date of publication of application:
**24.10.90 Bulletin 90/43**

(84) Designated Contracting States:
**AT BE CH DE ES FR GB GR IT LI LU NL SE**

(71) Applicant: **Warner, Glenfield**
**3010 Matis Street**
**St. Laurent Québec H4R 1A3(CA)**

(72) Inventor: **Warner, Glenfield**
**3010 Matis Street**
**St. Laurent Québec H4R 1A3(CA)**

(74) Representative: **Casalonga, Axel et al**
**BUREAU D.A. CASALONGA - JOSSE**
**Morassistrasse 8**
**D-8000 München 5(DE)**

(54) **Heart-related parameters monitoring apparatus.**

(57) A non-invasive method, and an apparatus, for determining heart-related parameters in patients. The method and apparatus determine pulse pressure, time constant of the arterial system, systolic and diastolic pressure, peripheral resistance, cardiac output and mean arterial blood pressure.

FIG. 1

EP 0 393 228 A1

## HEART-RELATED PARAMETERS MONITORING APPARATUS

### BACKGROUND OF INVENTION

Field of the Invention

The invention relates to a non-invasive method of measuring arterial blood pressure and cardiac output. The invention also relates to an apparatus for carrying out the method.

Description of Prior Art

Non-invasive methods an apparatus for measuring arterial blood pressure and cardiac output are known in the art. One such method and apparatus is illustrated in U.S. Patent 4,030,485, Warner, issued June 21, 1977. A second such method and apparatus is taught in U.S. Patent 4,418,700, Warner, issued December 6, 1983. The present invention constitutes an improvement and refinement of the method and apparatus as taught in the latter patent.

### SUMMARY OF INVENTION

The invention relates to a non-invasive method, and an apparatus for determining heart-related parameters in patients. The method and apparatus determine pulse pressure, time constant of the arterial system, systolic and diastolic pressure, peripheral resistance, and cardiac output and mean arterial blood pressure.

### BRIEF DESCRIPTION OF DRAWINGS

The invention will be better understood by an examination of the following description together with the accompanying drawings in which:

FIGURE 1 is a block diagram of the apparatus for carrying out the inventive method;

FIGURE 2 is a typical sensor output of the system as illustrated in Figure 1;

FIGURE 3 illustrates arterial blood pressure pulses;

FIGURES 4, 4a and 4b illustrate a blood volume pulse;

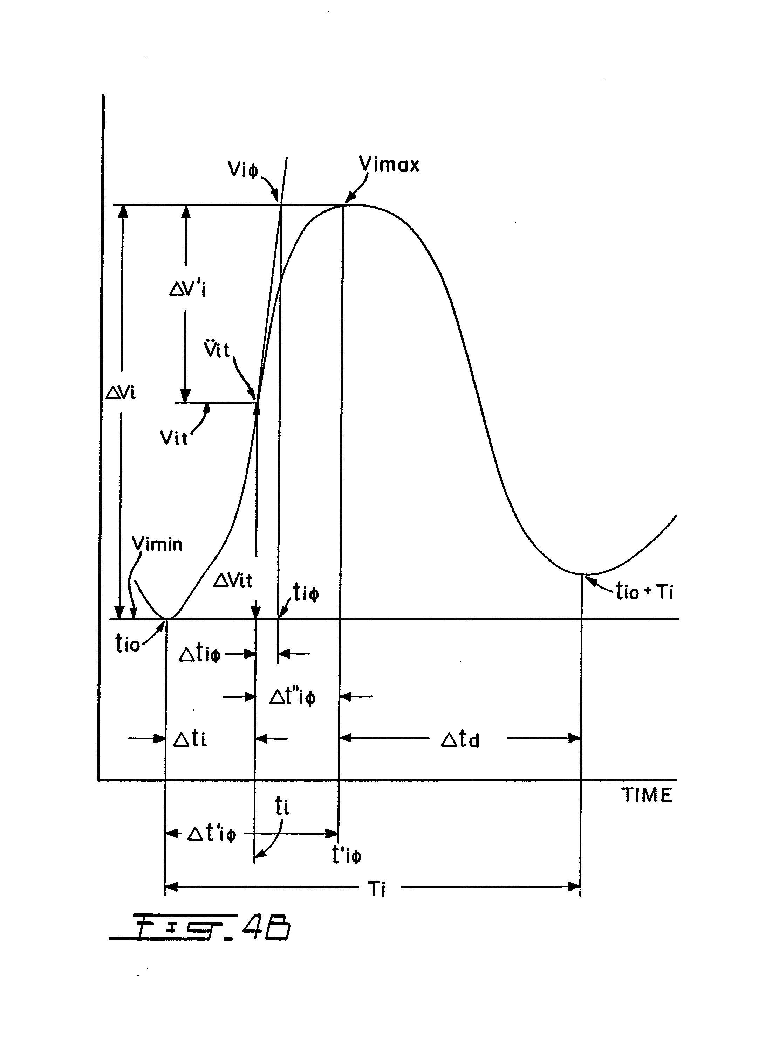

FIGURE 5 illustrates a blood volume pulse and a blood pressure pulse to illustrate the ratio g; and

FIGURE 6 is a simplified flowchart for a computer program for performing calculations in accordance with the invention.

### DESCRIPTION OF PREFERRED EMBODIMENTS

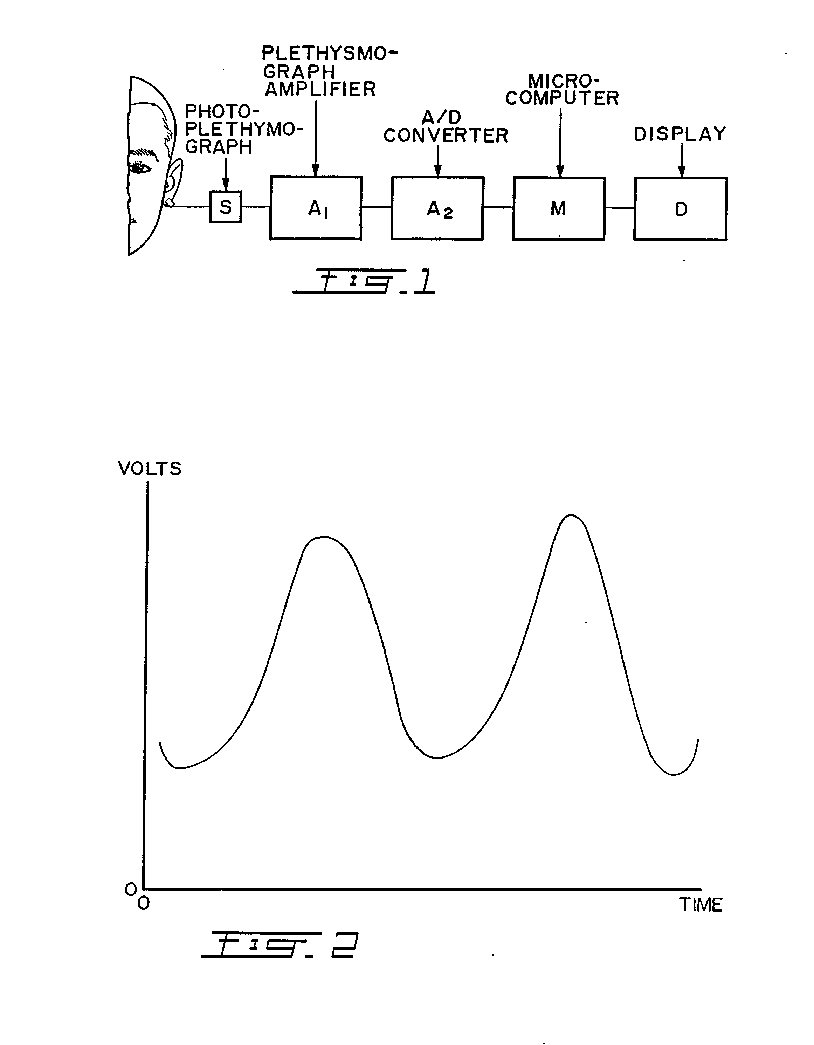

As seen in Figure 1, an apparatus in accordance with the invention comprises a volume sensor such as a photo-electric plethysmograph S, an amplifier $A_1$, an analog to digital converter $A_2$, a microcomputer M and a display device D. The plethysmograph sensor S is attached to, for example, the earlobe of a subject. The sensor could also be attached to other suitable parts of the body such as the forehead, fingertips or toes.

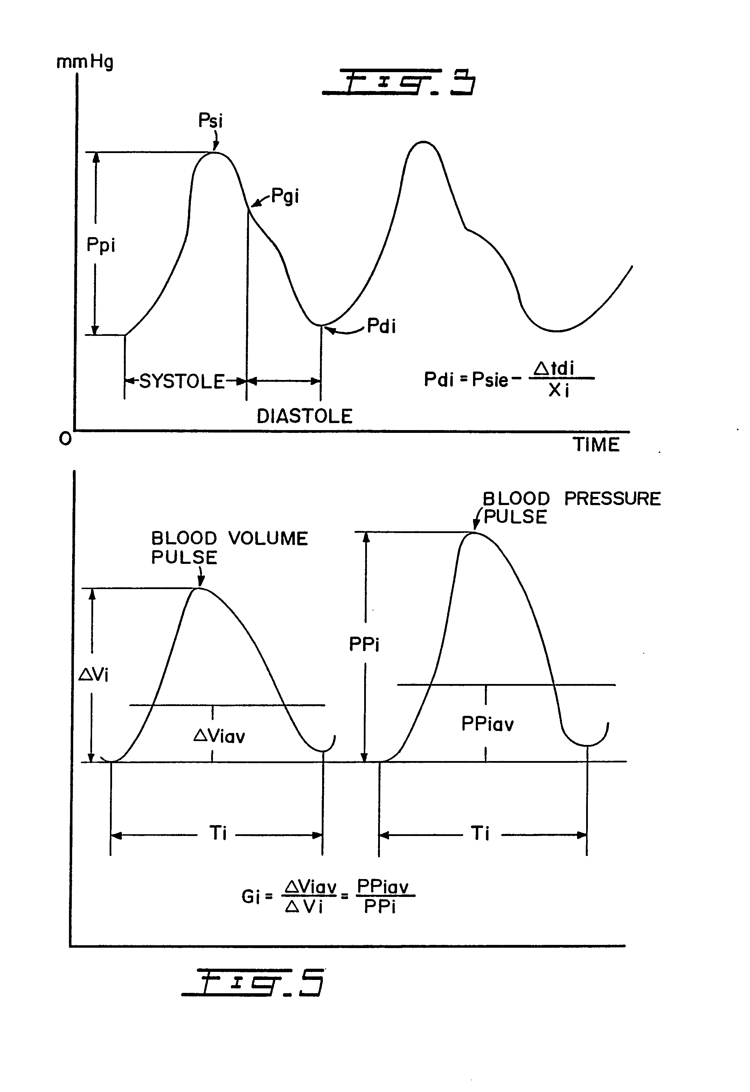

As is known, the plethysmograph detects changes in blood volume of the region to which it is attached. A typical sensor output signal is shown in Figure 2. As seen in Figure 2, the output signal has a pulsating component and a DC component.

The output of the sensor is applied to the plethysmograph amplifier $A_1$ where it is amplified and filtered and the DC component is discarded. The output of $A_1$ has a DC component, but this is not directly related to the sensor DC component.

The output of $A_1$ is fed to the analog to digital (A/D) converter $A_2$ which digitizes the signal. In a preferred embodiment, the sampling rate is 100 per second.

Microcomputer M accepts signals from $A_2$ and processes them according to the instructions it contains.

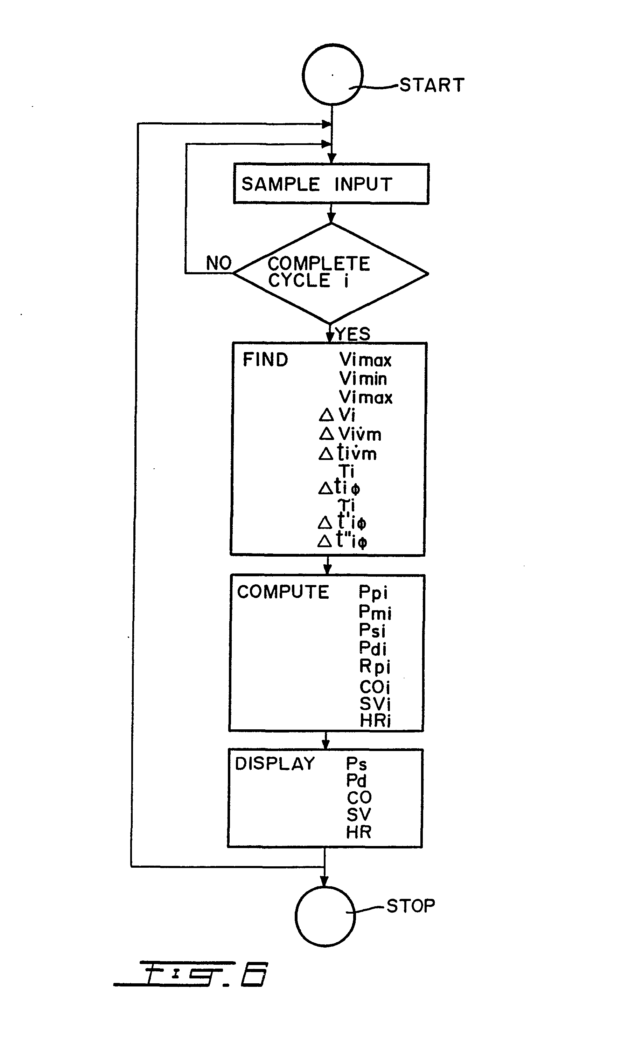

These instructions are schematically represented in the simplified flowchart of Figure 6.

The computer quantitites are then displayed on a CRT monitor D or other suitable display means.

## THEORY

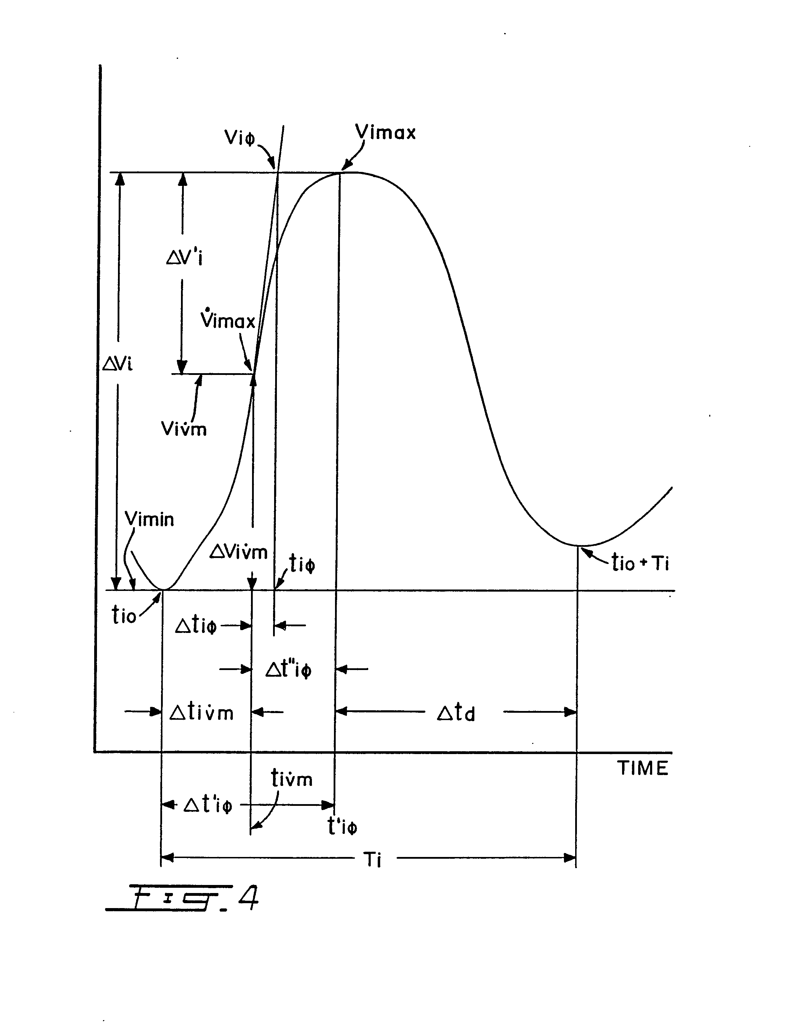

Arterial blood pressure pulses are shown in Figure 3. The shape of these curves vary according to the site where they are measured. The highest pressure reached during a cycle i is called the arterial systolic blood pressure, $P_{si}$. The lowest pressure reached during the same cycle is called the arterial diastolic blood pressure, $P_{di}$. The pressure rise from $P_{di}$ to $P_{si}$ in the same cycle is the pulse pressure $P_{pi}$.

By definition $P_{si} - P_{di} = P_{pi}$      (1)

### To find $P_{pi}$

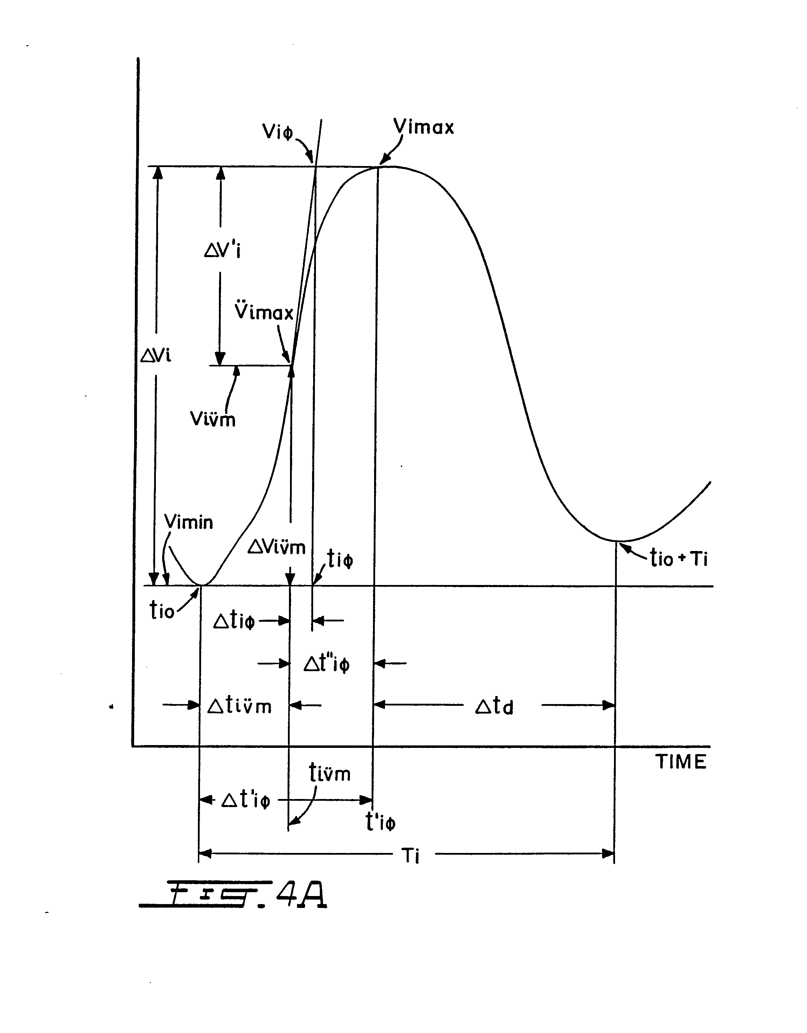

A plethysmographic pulse is shown in Figure 4. The minimum value at the beginning of the pulse is $V_{imin}$. The maximum value of the pulse is $V_{imax}$. As the pulse volume rises from $V_{imin}$ to $V_{imax}$, the time rate of volume change reaches a maximum $\dot{V}_{imax}$ at time $t_i \dot{v}_m$. The pulse volulme at time $V_i \dot{v}_m$ is $V_i \dot{v}_m$.

let

$$\frac{V_{i\dot{V}m} - V_{imin}}{V_{imax} - V_{imin}} = \frac{\Delta V_{i\dot{V}m}}{\Delta V_i} = R_i \qquad (2)$$

In addition to finding the values of $V_i \dot{v}_m$ corresponding to $\dot{V}_{imax}$, see U.S. Patent 4,418,700, Warner, values of $V_i \dot{v}_m$ are also found corresponding to $\dot{V}_{imax}$-1, $\dot{V}_{imax}$-2, ... $\dot{V}_{imax}$-k, where k is a function of $\dot{V}_{imax}$.

All of the values of $V_i \dot{v}_m$ corresponding to the time rates of volume change lying between and including $\dot{V}_{imax}$ and $\dot{V}_{imax}$-k are averaged and used to compute $\Delta V_i \dot{v}_m$.

The average value of $V_i \dot{v}_m$ is

$$\overline{V}_{i\dot{V}m} = \frac{\sum_1^{n0} V_{i\dot{V}_0 mn0} + \sum_1^{n1} V_{i\dot{V}_1 mn1} + \ldots + \sum_1^{nk} V_{i\dot{V}_k mnk}}{n0 + n1 + \ldots + nk}$$

where

n0 = number of values of $V_i \dot{v}_0 m$ corresponding to $\dot{V}_{imax}$

n1 = number of values of $V_i \dot{v}_1 m$ corresponding to $\dot{V}_{imax}$-1

... = ......................................... ..........

nk = number of values of $V_i \dot{v}_k m$ corresponding to $\dot{V}_{imax}$-k

$$k = \frac{\dot{V}_{imax}}{m} \text{ (integral values only)} + \ell$$

m = constant ... a preferred value of m = 20

$\ell$ = constant ... a preferred value of $\ell$ = 1

$$P_{pi} = K_{pp} \left( \frac{R_i - r_1}{(1 + r_2 - R_i)} \propto \right) \qquad (4a)$$

$K_{pp}$ = constant determined by a first calibration

$r_1$ = constant ... preferably equal to 0

$r_2$ = constant ... preferably equal to 0

$0 \leq \alpha \leq 1$

$R_i$ can now be defined, as per equation (2) above, but using the average value of $V_i$ $\dot{v}_m$ so that equation (2) can be rewritten

let

$$\frac{\overline{V}_{i\dot{V}m} - V_{imin}}{V_{imax} - V_{imin}} = \frac{\Delta V_{i\dot{V}m}}{\Delta V_i} = R_i \qquad (2)$$

from Figure 4

$\Delta V'_i = \Delta V_i - \Delta V_i \dot{v}_m$

$$\frac{\Delta V'_i}{\Delta V_i} = R' = \frac{\Delta V_i - \Delta V_{i\dot{v}m}}{\Delta V_i} = 1 - \frac{\Delta V_{i\dot{v}m}}{\Delta V_i} - 1 - R_i$$

wherein $R'_i = 1 - R_i$
or $R_i = 1 - R_i$

No other calibration should be required with different subjects. However, if desired, $K_{pp}$ can be determined for each subject.


To find mean blood pressure

The mean blood pressure $P_{mi}$ during a cycle $i$ is given by

$$P_{mmi} = K_4 \left[ \frac{\Delta \dot{V}_i}{\Delta \dot{V}_{imax}} \right]^{-b_3} \qquad (5)$$

$P_{mi} = P_{mmi} + P_0$  (6)
$b_3$ = exponent ... the preferred value of $b_3$ is equal to 0.5
$K_4$ = constant determined at calibration for each subject. It is only necessary to find this constant once for each subject. The measurements carried out at different times on the same subject do not require separate calibration
$P_0$ = constant ... preferred 25 mmHg
$\Delta \dot{V}_{imax}$ = maximum time rate of change of $\Delta V_i = \dot{V}_{imax}$
$P_{si} = P_{mi} + (1-g_i)P_{pi}$  (7)
where

$$g_i = \frac{\Delta V_{iAV}}{\Delta V_i} -$$

$\Delta V_{iAV}$ = average value of $\Delta V_i$ over the time interval $T_i$
$P_{di} = P_{si} - P_{pi}$  (8)
The variable $g_i$ can take on a constant value $g_0$ whose preferred value is 0.333.
Alternatively, mean blood pressure can be determined using the following expression:

$$P_{mmi} = \left[ K_4 \; \frac{\Delta V_i}{\Delta \dot{V}_{imax}[G(t)]} \right]^{-b_3} \qquad (5')$$

$$= K_4 \left( \frac{1}{r_i} \right)^{-b_3}$$

(for definition of $r_i$, see equation (10) below)
where
$G(t)$ = a function of t,
in a particular case,

$$G(t) = \frac{\phi_c}{\phi_i}$$

$$\phi_c = \left( \frac{1}{\Delta t_c} \right)^y$$

$$\phi_i = \left( \frac{1}{\Delta t_i} \right)^y$$

$\Delta t_i = \Delta t'_{i\phi}$
$\Delta t_c = \Delta t$ at calibration $= \Delta t'_{i\phi c}$ (see Figure 4B)
where
$T_c$ = T at calibration
$t_c$ = t at calibration

$$\phi_c = \frac{T_c}{t_c} = \frac{T}{t}$$

at calibration
$y$ = constant.
The remainder of the terms in equation the same as similar terms in equation

## Determination of Ratio R (Figure 4b)

From Figure 4b, the ratio R is

$$R_i = \frac{\Delta V_{it}}{\Delta V_i}$$

where
$\Delta V_{it}$ = change in volume at predetermined time ti
$\Delta V_i$ = total volume change during cycle i
$t_i$ = time such that $\Delta t_i = K_T \Delta t'_{i\phi}$
$K_T$ = constant

## Estimation of pulse pressure, PP

$$K_{100} \left( \frac{1 - e^{-K'_T kPP_i}}{1 - e^{-kPP_i}} \right) = R_i$$

where

$PP_i$ = pulse pressure = $P_s - P_d$

$P_s$ = systolic blood pressure

$P_d$ = diastolic blood pressure

$k$ = constant

$K'_T$ = constant $\simeq K_T$

$K_{100}$ = coefficient

In Figure 4B

$\Delta V'_i = \Delta V_i - \Delta V_{it}$

$$\frac{\Delta V'_i}{\Delta V_i} = \frac{\Delta V_i - \Delta V_{it}}{\Delta V_i} = 1 - \frac{\Delta V_{it}}{\Delta V_i} = 1 - R_i = R'_i$$

wherein from the above equation:

$$K_{100}\left( 1 - \frac{1 - e^{-K_T kPP_i}}{1 - e^{-kPP_i}} \right) = 1 - R_i$$

$$K_{100}\left( \frac{1 - e^{-kPP_i} - (1 - e^{-K_T kPP_i})}{1 - e^{-kPP_i}} \right) = 1 - R_i$$

$$K_{100}\left( \frac{- e^{-kPP_i} + e^{-K_T kPP_i}}{1 - e^{-kPP_i}} \right) = 1 - R_i$$

multiply numerator and denominator by $e^{kPP_i}$

$$K_{100}\left( \frac{-1 + e^{kPP_i} \, e^{-K_T kPP_i}}{e^{kPP_i} - 1} \right) = 1 - R_i$$

$$K_{100}\left( \frac{e^{kPP_i(1-K_T)} - 1}{e^{kPP_i} - 1} \right) = 1 - R_i = R'_i$$

Determination of r

From Figure 4

$$r_i = \frac{\dot{V}_{imax}}{\Delta V_i} \; G(t)$$

where
$\dot{V}_{imax}$ = maximum time rate of volume increase in cycle i
$\Delta V_i$ = total volume increase during cycle i

From Figure 4b

$$r = \frac{\dot{V}_{it}}{\Delta V_i} \; G(t)$$

where
$\dot{V}_{it}$ = time rate of increase of volume $V_{it}$ at time $t_i$
$\Delta V_i$ = total volume increase of volume during cycle i

Estimation of Mean Blood Pressure

1) $P'_{mi} = K_1 r_{i_c}^a$
where
$K_1$ = calibration constant

$$P'_{mi} = \frac{P_s + P_d}{2} - P_o$$

$P_{si}$ = systolic blood pressure, in cycle i

$$P_{mi} = \frac{P_s + P_d}{2}$$

$P_{di}$ = diastolic blood pressure, in cycle i
a = constant
$P_o$ = constant
2) $e^{kP_{mi}} = k_2 r_{i_c}^b$
where
$K_2$ = constant (calibration)
b = constant

$$K_3 \left( \frac{e^{-k(P_{mo} - j\emptyset_{1i})} - e^{-k(P_{mo} + j\emptyset_{2i})}}{e^{-k(P_{mo} - \emptyset_{1i})} - e^{-k(P_{mo} + \emptyset_{2i})}} \right) = r_i \qquad (9)$$

where
$P_{mo}$ = constant at calibration
$\emptyset_{1i} + \emptyset_{2i} = PP_i$ = pulse pressure during cycle i
k = constant
j = constant
$K_3$ = coefficient
solve equation by making LHS = RHS by varying $\emptyset_{1i}$ and $\emptyset_{2i}$ ($\emptyset_{2i} = PP_i - \emptyset_{1i}$)

then

$P_{si} = P_{mo} + \emptyset_{2i} + P_o$

$P_{di} = P_{mo} - \emptyset_{1i} + P_o$

$$P_{mi} = \frac{P_{si}' + P_{di}}{2}$$

$P_o$ = constant

$r_i$ = ratio of exponentials

$K_3$ = coefficient (variable or constant)

Correction for $r_i$

$$r_i \text{ (corrected)} = r_{ic} = r_i \ e^{m(\phi_o - \phi_i)}$$

$m$ = constant

$\emptyset_o$ = $PP_i$ at calibration

$\emptyset_i$ = current value of $PP_i$.

Equation (9) above is only one form which this particular equation can take. By simple mathematical manipulations, the invention may take two other forms as per (10) and (11) below. What follows is the manipulations as well as the two other forms of the equation:

As above noted

$\emptyset_{2i} + \emptyset_{1i} = PP_i = P_{si} - P_{di}$

$\emptyset_{2i} + \emptyset_{1i} = (P_{si} - P_o) - (P_{di} - P_o)$

Let $P_{si}' = P_{si} - P_o$

$P_{di}' = P_{di} - P_o$

$\emptyset_{2i} + \emptyset_{1i} = P_{si}' - p_{di}'$

add and subtract $P_{mo}$ on RHS above

$\emptyset_{2i} + \emptyset_{1i} = P_{si}' - P_{mo} + P_{mo} - P_{di}'$    (A)

$\emptyset_{2i}$ and $\emptyset_{1i}$ can take on any values in satisfying the above equation (A)

Put $\emptyset_{2i} = p_{si}' - P_{mo}$

and $\emptyset_{1i} = P_{mo} - P_{di}'$ in equation (9)

then

$$K_3 \left[ \frac{e^{-k(P_{mo} - j(P_{mo} - P'_{di}))} - e^{-k(P_{mo} + j(P'_{si} - P_{mo}))}}{e^{-k(P_{mo} - (P_{mo} - P'_{di}))} - e^{-k(P_{mo} + (P'_{si} - P_{mo}))}} \right] = r_i \ \ldots (10)$$

simplifying the denominator

$$K_3 \left[ \frac{e^{-k(P_{mo} - j(P_{mo} - P'_{di}))} - e^{-k(P_{mo} + j(P'_{si} - P_{mo}))}}{e^{-kP'_{di}} - e^{-kP'_{si}}} \right] = r_i \ \ldots (11)$$

To solve equation 11:

1) Set $P_{di}' = P_{si}' - PP_i$ and solve for $P_{si}'$

2) Set $P_{si}' = P_{di}' - PP_i$ and solve for $P_{di}'$

Although particular embodiments have been illustrated, this was for the purpose of describing, but not limiting, the invention. Various modifications, which will come readily to the mind of one skilled in the art, are within the scope of the invention as defined in the appended claims.

**Claims**

1. Apparatus for determining the magnitude of heart-related parameters in a patient; comprising;
means for detecting blood volume, and thereby blood volume variation, in said patient, and for providing a signal representative of said blood volume, and thereby said blood volume variation;
said means for detecting being attachable to said patient to thereby detect said blood volume, and thereby said blood volume variation;
said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change of said signal being representative of the difference in volume between the maximum amount of blood volume and the volume at the time of maximum rate of change of said blood volume, and a pulse repetition period;
means for measuring said maximum amplitude, said minimum aplitude, said maximum rate of change of said signal, said first difference, said second different, said first time interval, and said second time interval; and
means for calculating the magnitude of selected ones of said parameters, said means for calculating being connected to both said means for detecting and means for measuring;
wherein means for calculating calculates the magnitude of the pulse pressure parameter in accordance with the following expression:

$$P_{pi} = K_{pp} \left( \frac{R_{i1} + r_1}{(1 + r_2 - R_{i1})} \right)$$

wherein
$P_{pi}$ = pulse pressure during cycle i
$K_{pp}$ = constant determined by a first calibration
$r_1$ = constant
$r_2$ = constant

$$R_{i1} = \frac{\Delta V_i \dot{V}_m}{\Delta V_i}$$

where
$\Delta V_i \dot{V}_m$ = volume change at time $t_{i\dot{V}m}$ during cycle i corresponding to maximum rate of volume change, $V_{imax}$
$\Delta V_i$ = maximum volume change during cycle i cycle i
$\Delta t_i \dot{V}_m$ = time interval from start of cycle i to time of maximum rate of volume change $V_{imax}$.

2. Apparatus for determining the magnitude of heart-related parameters in a patient; comprising;
means for detecting blood volume, and thereby blood volume variation, in said patient, and for providing a signal representative of said blood volume, and thereby said blood volume variation;
said means for detecting being attachable to said patient to thereby detect said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change of said signal being representative of the difference in volume between the maximum amount of blood volume and the volume at the time of maximum rate of change of said blood volume, and a pulse repetition period;

means for measuring said maximum amplitude, said minimum aplitude, said maximum rate of change of said signal, said first difference, said second different, said first time interval, and said second time interval; and

means for calculating the magnitude of selected ones of said parameters, said means for calculating being connected to both said means for detecting and means for measuring;

wherein means for calculating calulates the magnitude of the mean arterial blood pressure, $P_{mi}$ parameter in accordance with the following expression:

$$P_{mi} = P_o + P_{mmi}$$

$$P_{mmi} = K_4 \left[ \frac{\Delta V_i}{\Delta \dot{V}_{imax} \, [G(t)]} \right]^{-b_3}$$

where

$K_4$ = constant determined for each subject

$b_3$ = constant

$P_{mmi}$ = pseudo mean arterial blood pressure during cycle i

$\Delta V_i$ = maximum volume change during cycle i

$\Delta \dot{V}_{imax}$ = maximum time rate of change of $\Delta V_i$

$= \dot{V}_{imax}$

where

$$G(t) = \frac{\phi_c}{\phi_i}$$

$$\phi_c = \left[ \frac{1}{\Delta t_c} \right]^y$$

$$\phi_i = \left[ \frac{1}{\Delta t_i} \right]^y$$

where

$\Delta t_c$ = $\Delta t$ at calibration = $\Delta t_{i\phi c}$

$\Delta t_i$ = $\Delta t_{i\phi}$

y = constant.

3. Apparatus for determining the magnitude of heart-related parameters in a patient; comprising;

means for detecting blood volume, and thereby blood volume variation, in said patient, and for providing a signal representative of said blood volume, and thereby said blood volume variation;

said means for detecting being attachable to said patient to thereby detect said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change of said signal being representative of the difference in volume between the maximum amount of blood volume and the volume at the time of maximum rate of change of said blood volume, and a pulse repetition period;

means for measuring said maximum amplitude, said minimum aplitude, said maximum rate of change of said signal, said first difference, said second different, said first time interval, and said second time interval; and

means for calculating the magnitude of selected ones of said parameters, said means for calculating being connected to both said means for detecting and means for measuring;

wherein means for calculating calculates the magnitude of the systolic pressure ($P_{si}$) parameter in accordance with the following expression::

$$P_{si} = P_{mi} + (1-g_0)P_{pi}$$

wherein

$g_0$ = constant.

4. Apparatus for determining the magnitude of heart-related parameters in a patient; comprising;

means for detecting blood volume, and thereby blood volume variation, in said patient, and for providing a signal representative of said blood volume, and thereby said blood volume variation;

said means for detecting being attachable to said patient to thereby detect said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change of said signal being representative of the difference in volume between the maximum amount of blood volume and the volume at the time of maximum rate of change of said blood volume, and a pulse repetition period;

means for measuring said maximum amplitude, said minimum aplitude, said maximum rate of change of said signal, said first difference, said second different, said first time interval, and said second time interval; and

means for calculating the magnitude of selected ones of said parameters, said means for calculating being connected to both said means for detecting and means for measuring;

wherein means for calculating calculates the magnitude of the systolic pressure ($P_{si}$) parameter in accordance with the following expression:

$$P_{si} = P_{mi} + (1-g_0)P_{pi}$$

wherein:

$$g_i = \frac{\Delta V_{iAV}}{\Delta V_i}$$

$P_{pi}$ = pulse pressure during cycle i

$\Delta V_i$ = represented by said first difference

$\Delta V_{iAV}$ = represented by the difference between said minimum amplitude and an amplitude equal to the average value of a pulse in a cycle i.

5. Apparatus for determining the magnitude of heart-related parameters in a patient;
comprising;
means for detecting blood volume, and thereby blood volume variation, in said patient, and for providing a signal representative of said blood volume, and thereby said blood volume variation;
said means for detecting being attachable to said patient to thereby detect said blood volume, and thereby said blood volume variation;
said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change of said signal being representative of the difference in volume between the maximum amount of blood volume and the volume at the time of maximum rate of change of said blood volume, and a pulse repetition period;
means for measuring said maximum amplitude, said minimum apIitude, said maximum rate of change of said signal, said first difference, said second different, said first time interval, and said second time interval; and
means for calculating the magnitude of selected ones of said parameters, said means for calculating being connected to both said means for detecting and means for measuring;
wherein means for calculating calculates the magnitude of the arterial blood pressure, $P_{mi}$ parameter in accordance with the following expression:

$P_{mi} = P_o + P_{mmi}$

$$P_{mmi} = K_4 \left[ \frac{\Delta V_i}{\Delta \dot{V}_{imax} [G(t)]} \right]^{-b_3}$$

where

$K_4$ = constant determined for each subject

$b_3$ = constant

$P_{mmi}$ = pseudo mean arterial blood pressure during cycle i

$\Delta V_i$ = maximum volume change during cycle i

$\Delta \dot{V}_{imax}$ = maximum time rate of change of $V_i = \dot{V}_{imax}$

$P_o$ = constant

where

$G(t)$ = a function of t.

6. method for determining the magnitude of heart-related parameters in a patient;
comprising:
detecting blood volume, and thereby blood volume variation, in said patient and providing a signal representative of said blood volume, and thereby said blood volume variation;
said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and said minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period;
measuring said maximum amplitude, said minimum amplitude, said maximum rate of change of said signal, said first difference, said second difference, said first time interval, and said second time interval; and
calculating the magnitude of the pulse pressure parameter in accordance with the following expression:

$$P_{pi} = K_{pp} \left[ \frac{R_{i1} - r_1}{(1 + r_2 - R_{i1})} \right]$$

wherein

$P_{pi}$ = pulse pressure during cycle i

$K_{pp}$ = constant determined by a first calibration

$r_1$ = constant

$r_2$ = constant

$$R_{i1} = \frac{V_{iVm}}{V_i}$$

where $\Delta V_{i\ \dot{v}m}$ = volume change at time $t_{iVm}$ during cycle i corresponding to maximum rate of volume change, $V_{imax}$

$\Delta V_i$ = maximum volume change during cycle i

$\Delta t_{i\ \dot{v}m}$ = time interval from start of cycle i to time of maximum rate of volume change $V_{imax}$.

7. A method for determining the magnitude of heart-related parameters in a patient; comprising:

detecting blood volume, and thereby blood volume variation, in said patient and providing a signal representative of said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and said minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period; measuring said maximum amplitude, said minimum amplitude, said maximum rate of change of said signal, said first difference, said second difference, said first time interval, and said second time interval; and calculating the magnitude of the mean artial pressure $P_{mi}$ in accordance with the following expression:

$P_{mi} = P_o + P_{mm_I}$

$$P_{mmi} = K_4 \left[ \frac{\Delta V_i}{\Delta \dot{V}_{imax}[G(t)]} \right]^{-b_3}$$

where

$K_4$ = constant determined for each subject

$b_3$ = constant

$P_{mmi}$ = pseudo mean arterial blood pressure during cycle i

$\Delta V_i$ = maximum volume change during cycle i

$\Delta \dot{V}_{imax}$ = maximum time rate of change of $\Delta V_i = \dot{V}_{imax}$

where

$$G(t) = \frac{\phi_c}{\phi_i}$$

$$\phi_c = \left[\frac{1}{\Delta t_c}\right]^y$$

$$\phi_i = \left[\frac{1}{\Delta t_i}\right]^y$$

where

$\Delta t_c = \Delta t$ at calibration $= \Delta t_{i\phi c}$

$\Delta t_i = \Delta t_{i\phi}$

$y$ = constant.

8. A method for determining the magnitude of heart-related parameters in a patient; comprising:

detecting blood volume, and thereby blood volume variation, in said patient and providing a signal representative of said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and said minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period;

measuring said maximum amplitude, said minimum amplitude, said maximum rate of change of said signal, said first difference, said second difference, said first time interval, and said second time interval; and calculating the magnitude of the systolic pressure ($P_{si}$) parameter in accordance with the following expression:

$P_{si} = P_{mi} + (1-g_0)P_{pi}$

wherein

$g_0$ = constant.

9. A method for determining the magnitude of heart-related parameters in a patient; comprising:

detecting blood volume, and thereby blood volume variation, in said patient and providing a signal representative of said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and said minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period;

measuring said maximum amplitude, said minimum amplitude, said maximum rate of change of said signal, said first difference, said second difference, said first time interval, and said second time interval; and calculating the magnitude of the systolic pressure ($P_{si}$) parameter in accordance with the following expression:

$P_{si} = P_{mi} + (1-g_i)P_{pi}$

wherein:

$$g_i \quad = \quad \frac{\Delta V_{iAV}}{\Delta V_i}$$

$P_{pi}$ = pulse pressure during cycle i

$\Delta V_i$ = represented by said first difference

$\Delta V_{iAV}$ = represented by the difference between said minimum amplitude and an amplitude equal to the average value of a pulse in a cycle i.

10. A method for determining the magnitude of heart-related parameters in a patient; comprising:

detecting blood volume, and thereby blood volume variation, in said patient and providing a signal representative of said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and said minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period;

measuring said maximum amplitude, said minimum amplitude, said maximum rate of change of said signal, said first difference, said second difference, said first time interval, and said second time interval; and calculating the magnitude of the arterial blood pressure, $P_{mi}$ parameter in accordance with the following expression:

$P_{mi} = P_o + P_{mmi}$

$$P_{mmi} \quad = \quad K_4 \left[ \frac{\Delta V_i}{\Delta \dot{V}_{imax} \ [G(t)]} \right]^{-b_3}$$

where

$k_4$ = constant determined for each subject

$b_3$ = constant

$P_{mmi}$ = pseudo mean arterial blood pressure during cycle i

$\Delta V_i$ = maximum volume change during cycle i

$\Delta \dot{V}_{imax}$ = maximum time rate of change of $V_i$

= $V_{imax}$

$P_o$ = constant

$G(t)$ = a function of t and T.

11. Apparatus for determining the magnitude of heart-related parameters in a patient; comprising;

means for detecting blood volume, and thereby blood volume variation, in said patient, and for providing a signal representative of said blood volume, and thereby said blood volume variation;

said means for detecting being attachable to said patient to thereby detect said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change of said signal being representative of the difference in volume between the maximum amount of blood volume and the volume at the time of maximum rate of change of said blood volume, and a pulse repetition period;

means for measuring said maximum amplitude, said minimum aplitude, said maximum rate of change of said signal, said first difference, said second different, said first time interval, and said second time interval; and

means for calculating the magnitude of selected ones of said parameters, said means for calculating being connected to both said means for detecting and means for measuring;

wherein means for calculating calculates the magnitude of the pulse pressure parameter in accordance with the following expression:

$$P_{pi} = K_{pp} \left( \frac{R_{i1} - r_1}{(1 + r_2 - R_{i1})} \right)$$

wherein

$P_{pi}$ = pulse pressure during cycle i

$K_{pp}$ = constant determined by a first calibration

$r_1$ = constant

$r_2$ = constant

$$R_{i1} = \frac{\Delta V_{i\dot{V}m}}{\Delta V_i}$$

where

$\Delta V_{i \dot{V}m}$ = volume change at preselected time $t_{iVm}$ during cycle i

$\Delta V_i$ = maximum volume change during cycle i

$\Delta t_{i \dot{V}m}$ = time interval from start of cycle i to preselected time of $t_{iVm}$.

12. A method for determining the magnitude of heart-related parameters in a patient; comprising:

detecting blood volume, and thereby blood volume variation, in said patient and providing a signal representative of said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and said minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period;

measuring said maximum amplitude, said minimum amplitude, said maximum rate of change of said signal, said first difference, said second difference, said first time interval, and said second time interval; and calculating the magnitude of the pulse pressure parameter in accordance with the following expression:

$$P_{pi} = K_{pp} \left[ \frac{R_{i1} - r_1}{(1 + r_2 - R_{i1})} \right]$$

wherein

$P_{pi}$ = pulse pressure during cycle i

$K_{pp}$ = constant determined by a first calibration

$r_1$ = constant

$r_2$ = constant

$$R_{il} = \frac{\Delta V_{i\dot{V}m}}{V_i}$$

where

$\Delta V_{i\dot{V}m}$ = volume change at preselected time $t_{iVm}$ during cycle i

$\Delta V_i$ = maximum volume change during cycle i

$\Delta t_{iVm}$ = time interval from start of cycle i to predetermined time of $t_{iVm}$.

13. Apparatus for determining the magnitude of heart-related parameters in a patient; comprising;

means for detecting blood volume, and thereby blood volume variation, in said patient, and for providing a signal representative of said blood volume, and thereby said blood volume variation;

said means for detecting being attachable to said patient to thereby detect said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change of said signal being representative of the difference in volume between the maximum amount of blood volume and the volume at the time of maximum rate of change of said blood volume, and a pulse repetition period;

means for measuring said maximum amplitude, said minimum amplitude, said maximum rate of change of said signal, said first difference, said second different, said first time interval, and said second time interval; and

means for calculating the magnitude of selected ones of said parameters, said means for calculating being connected to both said means for detecting and means for measuring;

wherein the means for calculating calculates the magnitude of the mean pressure parameter in accordance with the following expression:

1) $P'_{mi} = K_1 r_{ic}^{a}$

where

$K_1$ = calibration constant

$$P'_{mi} = \frac{P_s + P_d}{2} - P_o$$

$P_{si}$ = systolic blood pressure, in cycle i

$$P_{mi} = \frac{P_s + P_d}{2}$$

$P_{di}$ = diastolic blood pressure, in cycle i

a = constant

$P_o$ = constant.

14. A method for determining the magnitude of heart-related parameters in a patient; comprising:

detecting blood volume, and thereby blood volume variation, in said patient and providing a signal representative of said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and said minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period; measuring said maximum amplitude, said minimum amplitude, said maximum rate of change of said signal, said first difference, said second difference, said first time interval, and said second time interval; and calculating the magnitude of mean pulse pressure in accordance with the following expression:

1) $P'_{mi} = K_1 r_{ic}^a$

where

$K_1$ = calibration constant

$$P'_{mi} = \frac{P_s + P_d}{2} - P_o$$

$P_{si}$ = systolic blood pressure, in cycle i

$$P_{mi} = \frac{P_s + P_d}{2}$$

$P_{di}$ = diastolic blood pressure, in cycle i

a = constant

$P_o$ = constant

15. Apparatus for determining the magnitude of heart-related parameters in a patient; comprising;

means for detecting blood volume, and thereby blood volume variation, in said patient, and for providing a signal representative of said blood volume, and thereby said blood volume variation;

said means for detecting being attachable to said patient to thereby detect said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said minimum amplitude and said maximum amplitude, a maximum rate of change of said signal being representative of the maximum rate of increase of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum rate of change of said signal being representative of the difference in volume between the maximum amount of blood volume and the volume at the time of maximum rate of change of said blood volume, and a pulse repetition period;

means for measuring said maximum amplitude, said minimum amplitude, said maximum rate of change of said signal, said first difference, said second different, said first time interval, and said second time interval; and

means for calculating the magnitude of selected ones of said parameters, said means for calculating being connected to both said means for detecting and means for measuring;

wherein the means for calculating calculates implicitly the magnitude of the mean pulse pressure in accordance with the following expression:

$$K_3 \left( \frac{e^{-k(P_{mo}-j\emptyset_{1i})} - e^{-k(P_{mo}+j\emptyset_{2i})}}{e^{-k(P_{mo}-\emptyset_{1i})} - e^{-k(P_{mo}+\emptyset_{2i})}} \right) = r_i$$

where

$P_{mo}$ = constant at calibration

$\emptyset_{1i} + \emptyset_{2i} = PP_i$ = pulse pressure during cycle i

k = constant

j = constant

$P_{si} = P_{mo} + \emptyset_{2i} + P_o$

$P_{di} = P_{mo} - \emptyset_{1i} + P_o$

$$P_{mi} = \frac{P_{si} + P_{di}}{2}$$

$P_o$ = constant

$r_i$ = ratio of exponentials

$K_3$ = coefficient (variable or constant).

16. A method for determining the magnitude of heart-related parameters in a patient; comprising:

detecting blood volume, and thereby blood volume variation, in said patient and providing a signal representative of said blood volume, and thereby said blood volume variation;

said blood volume variation being cyclic in nature whereby said signal comprises a cyclic curve having, in each cycle of variation, a variable slope, a maximum amplitude representative of the maximum amount of blood volume, a minimum amplitude representative of the minimum amount of blood volume, a first time interval between said maximum amplitude and aid minimum amplitude, a maximum rate of change of said signal being representative of the maximum rate of change of blood volume, a second time interval between the minimum amplitude and the time of the maximum rate of change of said signal, a first difference in amplitude between said maximum amplitude and said minimum amplitude, a second difference in amplitude between the maximum amplitude and the amplitude at the time of maximum change of rate of said signal being representative of the difference in volume between the maximum amount of blood volume and the volume at the time of maximum rate of change of said blood volume, and a pulse repetition period; measuring said maximum amplitude, said minimum amplitude, said maximum rate of change of said signal, said first difference, said second difference, said first time interval, and said second time interval; and wherein the means for calculating calculates implicitly the magnitude of mean pulse pressure in accordance with the following expressure:

$$K_3 \left( \frac{e^{-k(P_{mo}-j\emptyset_{1i})} - e^{-k(P_{mo}+j\emptyset_{2i})}}{e^{-k(P_{mo}-\emptyset_{1i})} - e^{-k(P_{mo}+\emptyset_{2i})}} \right) = r_i$$

where

$P_{mo}$ = constant at calibration

$\emptyset_{1i} + \emptyset_{2i} = PP_i$ = pulse pressure during cycle i

k = constant

j = constant

$P_{si} = P_{mo} + \emptyset_{2i} + P_o$

$P_{di} = P_{mo} - \emptyset_{1i} + P_o$

$$P_{mi} = \frac{P_{si} + P_{di}}{2}$$

19

$P_o$ = constant
$r_i$ = ratio of exponentials
$k_3$ = coefficient (variable or constant).

PLETHYSMO-
GRAPH
AMPLIFIER

PHOTO-
PLETHYMO-
GRAPH

MICRO-
COMPUTER

A/D
CONVERTER

DISPLAY

S | A₁ | A₂ | M | D

FIG.1

VOLTS

0

0

TIME

FIG.2

EP 0 393 228 A1

$$Pdi = Psie - \frac{\Delta tdi}{Xi}$$

FIG.3

$$Gi = \frac{\Delta Viav}{\Delta Vi} = \frac{PPiav}{PPi}$$

FIG.5

Fig.4

$$\text{FIG.}\ 4A$$

$$Fig. 4B$$

START

SAMPLE INPUT

NO — COMPLETE CYCLE i

YES

FIND Vimax
Vimin
V̇imax
△Vi
△Viv̇m
△tiv̇m
Ti
△tiφ
Ŧi
△t'iφ
△t"iφ

COMPUTE Ppi
Pmi
Psi
Pdi
Rpi
COi
SVi
HRi

DISPLAY Ps
Pd
CO
SV
HR

STOP

*FIG. 6*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 160 994 (S. WARNER et al.) <br> * claims 1,3-6,9-11,14-17; figure 1 * <br> --- | 1-12 | A 61 B 5/02 <br> G 06 F 15/20 |
| A | GB-A-1 538 695 (BIOTRON MEDICAL PRODUCTS LTD.) <br> * page 20, line 30 - page 21, line 12 * <br> --- | 1 | |
| A | US-A-4 667 680 (D. M. ELLIS) <br> * abstract; figure 3 * <br> --- | 1 | |
| A,D | EP-A-0 060 116 (S. WARNER et al.) <br> * page 1, line 23 - page 2, line 1; figures 1,2 * <br> ----- | 1 | |

TECHNICAL FIELDS SEARCHED (Int. Cl.5)

A 61 B 5/00
G 06 F 15/00

The present search report has been drawn up for all claims

| Place of search | Date of completion of the search | Examiner |
|---|---|---|
| BERLIN | 29-11-1989 | WEIHS J.A. |